# EUROPÄISCHE PATENTSCHRIFT

(11) **EP 0 786 262 B1**
(45) Veröffentlichungstag und Bekanntmachung des Hinweises auf die Patenterteilung: **07.06.2000**
(21) Anmeldenummer: 97103776.7
(22) Anmeldetag: 15.02.1996
(51) Int. Cl.: A61M 1/32, A61M 1/36

(54) **Steckspitze zur Verwendung in einer Vorrichtung zur Behandlung von Blut**
Connecting needle for use with a blood treatment device
Aiguille de connection pour l'utilisation avec un dispositif de traitement du sang

(30) Priorität: 22.02.1995 DE 19506163
(43) Veröffentlichungstag der Anmeldung: 30.07.1997
(62) Teilanmeldung aus: 96102297.7
(73) Patentinhaber: Müller, Hans, 81547 München (DE)
(72) Erfinder: Müller, Hans, 81547 München (DE)
(74) Vertreter: Fleuchaus, Leo, Dipl.-Ing.

(56) Entgegenhaltungen:
- DE-A- 4 410 411
- DE-U- 8 901 513
- US-A- 2 887 107
- US-A- 3 325 641
- DATABASE WPI Section PQ, Week 8716 Derwent Publications Ltd., London, GB; Class P34, AN 87-114052 XP002017588 & SE 8 504 062 A (GAMBRO CARDIO AB) , 3.März 1987

## Beschreibung

Die Erfindung betrifft eine Steckspitze zur Verwendung in einer Vorrichtung zur extrakorporalen Behandlung von einem menschlichen oder tierischen Körper entnommenen Blut, wobei die Steckspitze in eine von der Vorrichtung umfaßten Vakuumflasche einführbar ist, und Anschlußmöglichkeiten für eine von der Vorrichtung umfaßte Gasleitung sowie für einen von der Vorrichtung umfaßten Blutschlauch aufweist.

Zur Erläuterung des der vorliegenden Erfindung zugrundeliegenden Problems soll zunächst die Arbeitsweise derart bekannter Vorrichtungen zur extrakorporalen Behandlung von Blut erläutert werden:

Die sauerstoffhaltigen Behandlungsmedien werden nachfolgend mit dem Begriff "Gasbehandlung" bezeichnet. Bei dieser Behandlung wird das abgenommene Blut z. B. in einer Vakuumflasche oder einem Kunststoffbeutel der Gas-behandlung ausgesetzt und anschließend wieder in das menschliche oder tierische Blutgefäß retransfundiert. Eine solche Vorrichtung geht aus den DE-OS 4238884 und 4340730 hervor und dient der Sauerstoffanreicherung im Blut. Eine Einrichtung zur Gasbehandlung von Blut mittels der hämatogenen Oxidationstherapie (HOT) ist durch die DE-OS 3521802 bekannt, wobei das Blut bei der Entnahme in ein Auffanggefäß abgepumpt wird, in welches anschließend durch Austausch eine Vorrichtung zur HOT-Behandlung eingesetzt wird. Durch diese Offenbarung ist es bekannt, das Blut im Auffanggefäß durch Zufuhr von Sauerstoff aufzuschäumen und mit Hilfe des Überdrucks durch die Sauerstoffzufuhr durch eine Quarzglasküvette in ein weiteres Auffanggefäß zu drücken, wobei das aufgeschäumte Blut einer UV-Bestrahlung ausgesetzt wird. Aus dem weiteren Auffanggerät wird das Blut anschließend wieder retransfundiert.

In der DE-OS 4410411 wurde eine Vorrichtung zur extrakorporalen Behandlung von Blut vorgeschlagen, bei der anstelle einer Vakuumflasche ein flexibler Blutbeutel Verwendung findet, der eine sehr einfache Handhabung der HOT-Behandlung ermöglicht und ein komplett montiertes Retransfusionsset zur Verfügung stellt, das in seiner Gesamtheit in einer verhältnismäßig kleinen Packung steril verpackt und zum Einsatz gebracht werden kann.

Aus der DE-OS 4410411 ist es weiterhin bekannt, Blut über eine separate Zuleitung in den aus einem weichen Kunststoffmaterial bestehenden Blutbeutel einzuführen und dort ebenfalls mit über eine weitere Zuleitung zugeführten Sauerstoff aufzuschäumen, wobei am Ende der Sauerstoffzuleitung eine Feinperleinrichtung vorgesehen ist, um eine möglichst optimale Aufschäumung des Bluts zu erzielen.

Da bei der Blutabnahme gemäß der DE-44 10 411 kein Unterdruck Verwendung findet, wird für die Blutabnahme verhältnismäßig viel Zeit benötigt.

Deshalb wird - nach wie vor - häufig zur Verwendung einer Vakuumflasche gegriffen, um die Blutabnahme mit Hilfe des Vakuums zu beschleunigen.

Der Erfindung liegt deshalb die Aufgabe zugrunde, eine Vorrichtung bereitzustellen, mit der auf eine konstruktiv möglichst einfache Art und Weise sowohl Blut als auch Gas in eine Vakuumflasche eingebracht werden können.

Diese Aufgabe wird durch die Maßnahmen des Anspruchs 1 gelöst. Weitere vorteilhafte Ausgestaltungen der Erfindung sind Gegenstand von Unteransprüchen.
Insbesondere kann durch eine spezielle Ausgestaltung der erfindungsgemäßen Steckspitze nach Anspruch 2 das für die Gasbehandlung zugeführte Gasmedium durch einen Diffusionskörper in das Blut eingeleitet werden.

Dadurch wird eine ausreichend starke Aufschäumung des Blutes in der Vakuumflasche gewährleistet.

Die Vorteile und Merkmale der Erfindung ergeben sich auch aus der nachfolgenden Beschreibung eines Ausführungsbeispieles in Verbindung mit den Zeichnungen; es zeigen:
- Fig. 1: ein Retransfusionsset mit einer ersten Ausführungsform einer erfindungsgemäßen Steckspitze;
- Fig. 2: das in Fig. 1 gezeigte Retransfusionsset mit einer weiteren Ausführungsform einer erfindungsgemäßen Steckspitze;
- Fig. 3: ein Schauglas mit einer dritten Ausführungsform einer erfindungsgemäßen Steckspitze mit integriertem Diffusionskörper;
- Fig. 4: eine vierte Ausführungsform einer erfindungsgemäßen Steckspitze mit integriertem Diffusionskörper.

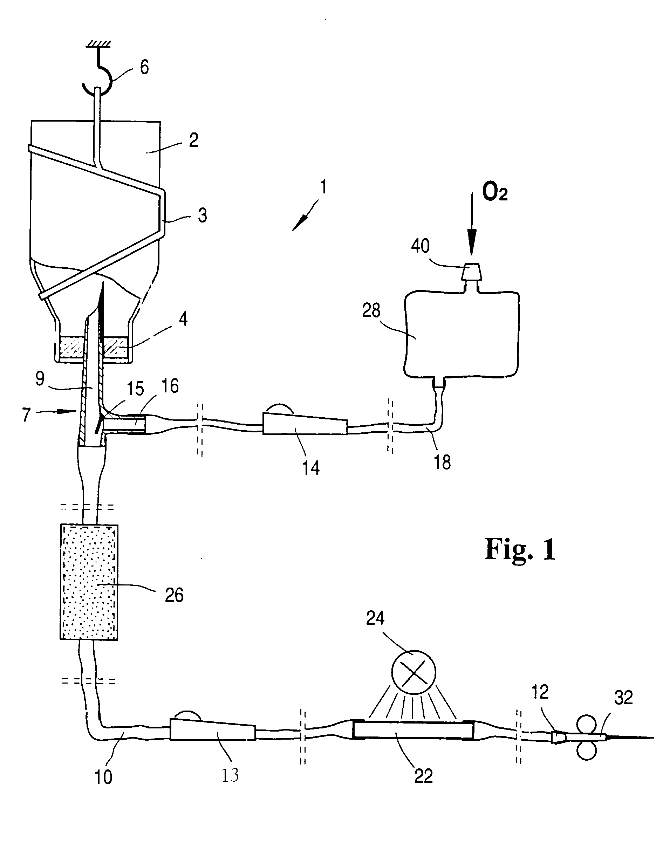

In Fig. 1 ist ein Retransfusionsset 1 für die Gas-Behandlung von Blut dargestellt, das mit Ausnahme der Vakuumflasche 2 als fertigmontiertes Besteck in steril versiegelten Blisterpackungen zur Verfügung steht. Die Vorrichtung besteht aus der Vakuumflasche 2, welche mit Hilfe einer Aufhängung 3 an einem Haken 6 aufgehängt werden kann. Durch den Gummistopfen 4 der Vakuumflasche ist eine Steckspitze 7 mit Kanüle 9 eingestochen. Die Kanüle 9 steht mit einem Blutfilter 26 sowie einem Gasanschluß 16 in Verbindung, der über ein Gasventil 15 in die Kanüle 9 mündet.

Hinter dem Blutfilter 26 führt in herkömmlicher Weise ein Blutschlauch 10 zu einer Quarzglasküvette 22 und von dort aus über ein Kupplungsstück 12 an eine Flügelkanüle 32. Zwischen der Quarzglasküvette 22 und dem Blutfilter 26 ist auf dem Blutschlauch 10 eine Rollklemme 13 montiert, mit welcher der Blutschlauch abgeklemmt werden kann. Die Quarzglasküvette 22 liegt im Strahlungsbereich einer UV-Lichtquelle 24.

An dem Gasanschluß 16 ist ein Gasschlauch 18 angeschlossen auf dem sich ebenfalls eine Rollklemme 14 zum Abquetschen des Gasschlauches befindet. Der Gasschlauch ist an dem dem Schauglas 8 gegenüberliegenden Ende an einen Druckausgleichsbeutel 28 angeschlossen, der seinerseits mit einer Kupplung 40 für den Anschluß einer Gasquelle versehen ist. Diese Gasquelle besteht in üblicher Weise aus einem unter hohem Druck stehenden Vorratsbehälter, von dem das Gasmedium über ein Reduzierventil dem Druckausgleichsbeutel zugeführt werden kann. Bei derartigen Reduzierventilen liegt der minimale Abströmdruck in der Regel zu hoch, so daß ein direktes Zuführen des Gasmediums in die Vakuumflasche in dieser einen hohen Druck aufbaut, der nur über ein zusätzliches Druckausgleichsventil vor dem Retransfundieren des Blutes abgebaut werden kann.

Durch die Verwendung des Druckausgleichsbeutels 28 läßt sich dieser unerwünschte hohe Druckaufbau in der Vakuumflasche vermeiden.

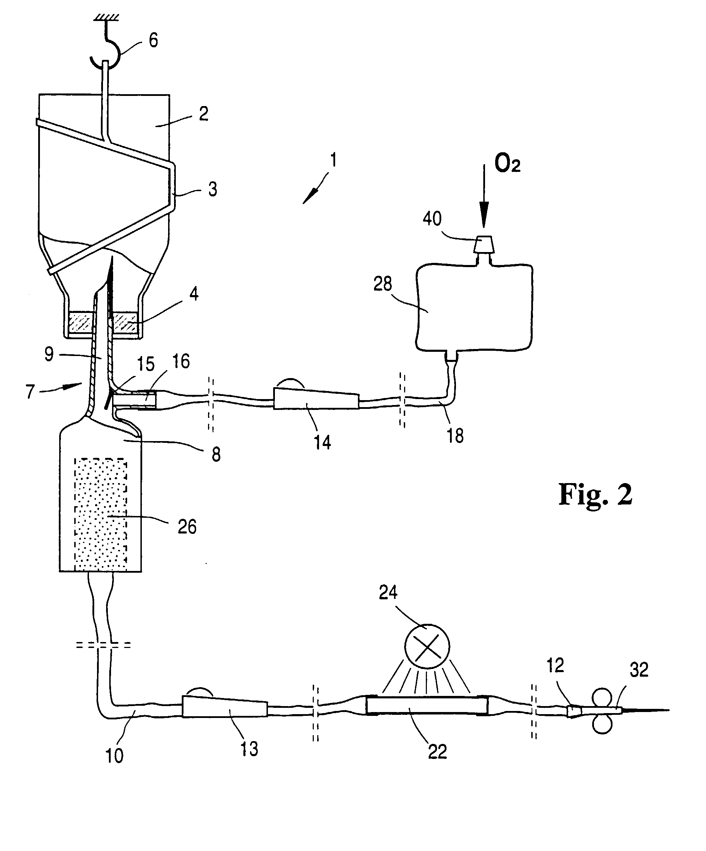

In Fig. 2 ist eine Ausführungsform der Erfindung dargestellt, bei der das Blutfilter 26 und die Steckspitze zu einer als Schauglas 8 ausgebildeten Einheit zusammengefaßt sind.

Eine Blutbehandlung mit Hilfe des Retransfusionssets läßt sich in der Regel wie folgt ausführen:

Zu Beginn der Behandlung wird das einteilige, aus mehreren Komponenten bestehende Retransfusionsset einer sterilen Verpackung entnommen und die Kanüle 9 der Steckspitze 7 durch den Gummistopfen 4 der Vakuumflasche 2 gestochen. Ferner wird die Kupplung 40 am Druckausgangsbeutel 28 an eine unter höherem Druck stehende Gasquelle angeschlossen, die beispielsweise eine Sauerstoffflasche mit Reduzierventil sein kann. Derartige Reduzierventile lassen sich auf einen minimalen Abströmdruck in der Größenordnung von etwa 5 bar einstellen, ein Druck, der für die einfache Handhabung des Retransfusionssets zu hoch ist.

Zum Abnehmen des Blutes wird mit der Flügelkanüle 32 ein Blutgefäß punktiert, und das Blut über den Blutschlauch 10, die geöffnete Rollklemme 13 und das Blutfilter 26 in die Vakuumflasche 2 gesaugt.

Zu Behandlung mit dem Gasmedium wird die Rollklemme 13 am Blutschlauch 10 geschlossen und die Rollklemme 14 am Gasschlauch 18 geöffnet. Damit kann die im Druckausgleichsbeutel 28 vorgegebene und gegenüber dem atmosphärischen Normaldruck etwas unter Überdruck stehende Luftmenge über den Gasanschluß 16 und das Gasventil 15 sowie die Kanüle 9 in die Vakuumflasche einströmen, wobei das Blut aufgeschäumt wird und sich das Vakuum in der Vakuumflasche abbaut. Wenn die im Druckausgleichsbeutel 28 vorhandene Luftmenge etwa der Menge entspricht, die für den Vakuumabbau benötigt wird, ergibt sich während der Gasbehandlung des Blutes ein Druckaufbau auf Normaldruck, so daß nunmehr für das Retransfundieren das Blut in der Vakuumflasche nahezu drucklos zur Verfügung steht. Zum Retransfundieren wird die Rollklemme 13 am Blutschlauch 10 geöffnet. Gleichzeitig bleibt auch die Rollklemme 14 am Gasschlauch 18 geöffnet, damit über den Druckausgleichsbeutel 28 ein Druckausgleich beim Retransfundieren erfolgt. Dazu dient entweder eine Restgasmenge im Druckausgleichsbeutel oder aber über das offene Kupplungsstück 40 zuströmende Luft. Beim Retransfundieren wird das durch die Quarzglasküvette 22 über die Flügelkanüle in das Gefäß zurückfließende und mit Sauerstoff aufgeschäumte Blut mit Hilfe der UV-Quelle 24 bestrahlt. Anstelle einer UV-Quelle kann auch eine Breitbandlichtquelle Verwendung finden.

Bei den in Fig. 1 und 2 gezeigten Steckspitzen ist der Wirkungsgrad des Aufschäumens noch mittels der Maßnahmen des abhängigen Anspruchs 2 verbesserungsfähig.

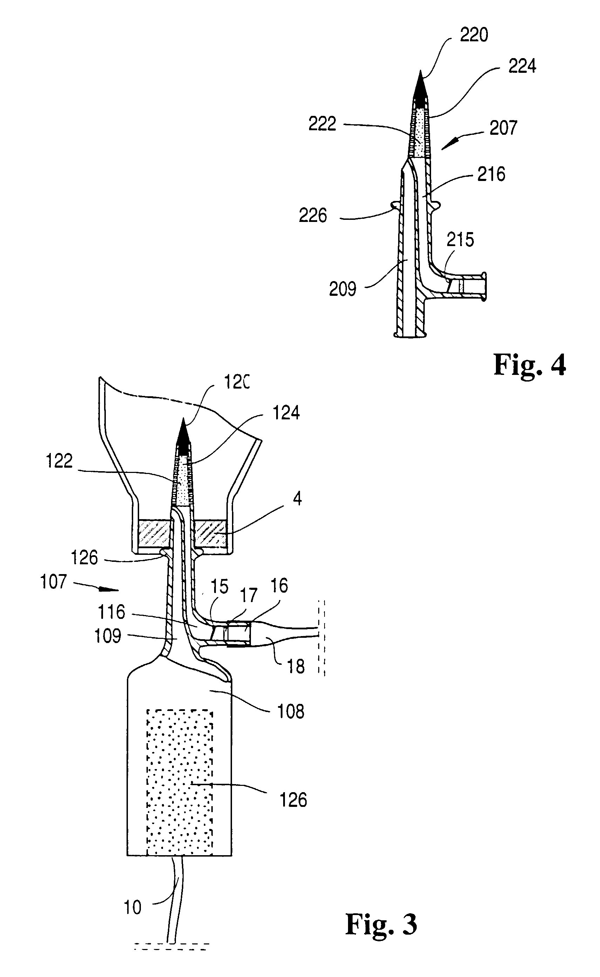

Fig. 3 zeigt eine erste Ausführungsform einer erfindungsgemäßen Steckspitze mit den Maßnahmen des Anspruchs 2.

Das in Fig. 3 gezeigte Schauglas 108 unterscheidet sich von einem herkömmlichen Schauglas dadurch, daß der Gasanschluß 16 in der erfindungsgemäßen Steckspitze 107 mit einer separaten Leitung 116 zur Spitze des Schauglases geführt wird. In dieser Spitze des Schauglases ist hinter einer eingegossenen Stahlspitze 120 ein Diffusionskörper 122 angeordnet und ferner ist der Bereich der Wandung des mit der Stahlspitze versehenen Dorns siebartig mit kleinen Löchern 124 versehen, so daß das über den Gasschlauch 18 zugeführte Gasmedium in feinster Verteilung in die Vakuumflasche 2 eingeleitet wird. Diese kleinen feinverteilten Gasbläschen erhöhen den Wirkungsgrad der Gasbehandlung ganz wesentlich.

Um sicherzustellen, daß beim Einstecken des Dorns in den Gummistopfen 4 die Austritts- und Eintrittsöffnung für das Blut an der Kanüle 109 sicher im Inneren der Vakuumflasche 2 zu liegen kommt, ist an dem Dorn ein Wulst 126 angebracht der beim Einstecken auf der Oberfläche des Gummistopfens zur Anlage kommen soll.

Schließlich ist in der Gasleitung 16 vorzugsweise im Bereich vor dem Gasventil 15 ein Bakterienfilter 17 vorgesehen.

In Fig. 4 ist eine erfindungsgemäße Steckspitze 207 gezeigt, die sich von der Ausführungsform nach Fig. 3 in ihrem Aufbau nicht wesentlich unterscheidet, jedoch nicht einstückig an ein Schauglas angeformt ist.

Durch die Integration des Diffusionskörpers in den Dorn des Schauglases 8 bzw. 108 ergibt sich eine viel zweckmäßigere Ausgestaltung des Retransfusionssets, wobei gleichzeitig die Kosten gegenüber herkömmlichen Diffusionsvorrichtungen verringert werden können.

Der wesentliche Vorteil der Erfindung wird darin gesehen, daß es auf Grund der erfindungsgemäßen Maßnahmen ermöglicht wird, Blut und Gas mit einem konstruktiv kompakten und einteiligen Anschlußstück in Form einer Steckspitze in ein Vakuumgefäß 2 einzuleiten und dort das über den Gasschlauch 18 zugeführte Gasmedium in feinster Verteilung in die Vakuumflasche 2 einzuleiten. Diese kleinen feinverteilten Gasbläschen erhöhen den Wirkungsgrad der Gasbehandlung ganz wesentlich.

Dabei kann die erfindungsgemäße Steckspitze nicht nur mit der in dem oben erwähnten Retransfusionsset zur Behandlung von Blut verwendet werden, sondern auch mit anderen derartigen Vorrichtungen, bei denen entnommenes Blut in einem Vakuumgefäß aufzuschäumen ist, um einen möglichst hohen Wirkungsgrad der Gasbehandlung zu erzielen.

## Patentansprüche

1. Steckspitze (7, 107, 207) zur Verwendung in einer Vorrichtung zur extrakorporalen Behandlung von einem menschlichen oder tierischen Körper entnommenen Blut, wobei die Steckspitze in eine von der Vorrichtung umfaßte Vakuumflasche (2) einführbar ist, und Anschlußmöglichkeiten für eine von der Vorrichtung umfaßte Gasleitung (18) sowie für einen von der Vorrichtung umfaßten Blutschlauch (10) aufweist, die über die Steckspitze in die Vakuumflasche einmünden, in der das Blut aufzuschäumen ist, wobei die Steckspitze mit einer an die Gasleitung (18) anschließbaren Kanüle (116, 216) und einer davon zumindest abschnittsweise getrennt geführten Kanüle (109, 209) für die Blutführung versehen ist.

2. Steckspitze nach Anspruch 1,
**dadurch gekennzeichnet,**
daß in der in die Vakuumflasche (2) einführbaren Spitze der Steckspitze hinter einer eingegossenen Spitze (120, 220) ein Diffusionskörper (122, 222) angeordnet ist, wobei der Bereich der Wandung um den Diffusionskörper siebartig mit kleinen Löchern versehen ist.

3. Steckspitze nach Anspruch 1 oder 2,
**dadurch gekennzeichnet**,
daß die Kanüle (109, 209) für die Blutführung hinter dem Diffusionskörper aus dem Schaft der Steckspitze austritt.

4. Steckspitze nach einem der vorstehenden Ansprüche,
**dadurch gekennzeichnet,**
daß die Spitze (120, 220) aus Stahl gefertigt ist.

5. Steckspitze nach einem der vorstehenden Ansprüche,
**dadurch gekennzeichnet,**
daß die Steckspitze in ihrem hinteren Teil mit einem Wulst (126, 226) versehen ist, der in einem vorgegebenen Abstand zur Mündung der Kanüle (109, 209) für die Blutführung im Schaft um die Steckspitze derart verläuft, daß die Mündung der Kanüle (109, 209) für die Blutführung beim Einstecken der Steckspitze in einen Gummistopfen (49) im Inneren der Vakuumflasche und der Wulst (126, 226) außen an dem Gummistopfen zur Anlage kommen.

6. Steckspitze nach einem der vorstehenden Ansprüche,
**dadurch gekennzeichnet,**
daß die Steckspitze ein Bakterienfilter (17) in der an die Gasleitung (18) anschließbaren Kanüle (116) enthält.

7. Steckspitze nach einem der vorstehenden Ansprüche,
**dadurch gekennzeichnet,**
daß die Steckspitze ein Gasventil (15) in der an die Gasleitung (18) anschließbaren Kanüle (116) enthält.

## Claims

1. Insertion spike (7, 107, 207) for utilisation with a device for extra-corporal treatment of blood from a human body or an animal, with the insertion spike being adapted for piercing a vacuum bottle being part of the device, said insertion spike being adapted for connecting to a gas hose (18) as well as to as blood-conducting conduit (10), which discharges via the insertion spike into the vacuum bottle for foaming the blood, said insertion spike comprising a passage (116, 216) connectable to the gas hose (18) and a passage (109, 209) separate from the other passage (116, 216) for conducting the blood.

2. Insertion spike according to claim 1,
**characterized in**
that the insertion spike insertable into the vacuum bottle comprises behind a cast-in-tip (120, 220) a diffusion body (122, 222) being perforated like a sieve with small holes with the wall area around the diffusion body.

3. Insertion spike according to claim 1 or 2
**characterized in**
that the passage (109, 209) for conducting the blood opens behind the diffusion body from the shaft of the insertion spike.

4. Insertion spike according to one of the preceeding claims,
**characterized in**
that the cast-in tip (120, 220) is made of steel.

5. Insertion spike according to one of the preceeding claims,
**characterized in that**
the insertion spike is provided with a shoulder (126, 226) disposed at a predetermined distance from the opening of the passage (109, 209) for conducting the blood such that, when the insertion spike has pierced a closure plug (49) and said shoulder abuts the closure plug said opening of the passage (109, 209) for conducting blood is dispersed within the vacuum bottle.

6. Insertion spike according to one of the preceeding claims,
**characterized in**
that the insertion spike comprises a bacteria filter (17) within the passage (116) connecting to the gas hose (18).

7. Insertion spike according to one of the preceeding claims,
**characterized in**
in that the insertion spike comprises a gas valve (15) within the passage (116) connecting to the gas hose (18).

## Revendications

1. Aiguille de raccordement (7, 107, 207) à utiliser dans un dispositif pour le traitement extracorporel de sang prélevé d'un corps humain ou animal, l'aiguille de raccordement pouvant être introduite dans une bouteille sous vide (2) comprise dans le dispositif, et présentant des possibilités de raccordement pour une conduite de gaz (18) comprise dans le dispositif et pour un flexible de sang (10) compris dans le dispositif, qui débouchent au moyen de l'aiguille de raccordement dans la bouteille sous vide où le sang doit être écumé, l'aiguille de raccordement étant pourvue d'une canule (116, 216) pouvant être raccordée à la conduite de gaz (18) et d'une canule (109, 209) guidée au moins pour partie séparément de la précédente, pour la conduite du sang.

2. Aiguille de raccordement selon la revendication 1, **caractérisée** en ce qu'un corps de diffusion (122, 222) est disposé en arrière d'une extrémité pointue moulée (120, 220) dans la pointe de l'aiguille de raccordement pouvant être introduite dans la bouteille sous vide (2), la région de paroi entourant le corps de diffusion étant pourvue de petits trous, à la manière d'un tamis.

3. Aiguille de raccordement selon la revendication 1 ou 2, **caractérisée** en ce que la canule (109, 209) pour la conduite du sang sort du corps de l'aiguille de raccordement en arrière du corps de diffusion.

4. Aiguille de raccordement selon une des revendications précédentes, **caractérisée** en ce que l'extrémité pointue (120, 220) est réalisée en acier.

5. Aiguille de raccordement selon une des revendications précédentes, **caractérisée** en ce que l'aiguille de raccordement est pourvue dans sa partie arrière d'un bourrelet (126, 226) qui s'étend autour de l'aiguille de raccordement, à une distance prédéfinie de l'embouchure dans le corps de l'aiguille de la canule (109, 209) pour la conduite du sang, de telle sorte que, lors de l'enfoncement de l'aiguille de raccordement dans un bouchon en caoutchouc (4) à l'intérieur de la bouteille sous vide, l'embouchure de la canule (109, 209) pour la conduite du sang et le bourrelet (126, 226) viennent s'appliquer extérieurement contre le bouchon en caoutchouc.

6. Aiguille de raccordement selon une des revendications précédentes, **caractérisée** en ce que l'aiguille de raccordement contient un filtre bactérien (17) dans la canule (116) pouvant être raccordée à la conduite de gaz (18).

7. Aiguille de raccordement selon une des revendications précédentes, **caractérisée** en ce que l'aiguille de raccordement contient une soupape de gaz (15) dans la canule (116) pouvant être raccordée à la conduite de gaz (18).
